# EUROPEAN PATENT SPECIFICATION

(11) **EP 4 017 489 B1**
(45) Date of publication and mention of the grant of the patent: **07.05.2025**
(21) Application number: 20854314.0
(22) Date of filing: 20.08.2020
(51) Int. Cl.: A61K 31/4164, A61P 35/00, C07D 235/00, A61K 31/4178, C07D 471/10

(54) **DGD1202 FOR USE IN THE TREATMENT OF KRAS-MUTATED CANCERS**
DGD1202 ZUR BEHANDLUNG VON KRAS-MUTIERTEN KREBSEN
DGD1202 POUR LE TRAITEMENT DES CANCERS À MUTATION KRAS

(30) Priority: 22.08.2019 US 201962890119 P
(43) Date of publication of application: 29.06.2022
(73) Proprietor: The Regents Of The University Of Michigan, Ann Arbor, MI 48109-2590 (US)
(72) Inventor: NYATI, Mukesh, K., Superior Township, MI 48198 (US)
(74) Representative: Zacco Sweden AB
(86) International application number: PCT/US2020/047090
(87) International publication number: WO 2021/034992

(56) References cited:
- WO-A1-2019/165358
- WO-A2-2014/176475
- US-A1- 2012 041 070
- US-A1- 2013 237 535
- US-A1- 2015 197 814
- DATABASE PubChem Compound 2 November 2019 (2019-11-02), "Schembl21312926 | C31H32BrN7OS", XP055794904, Database accession no. 139449533

## Description

### Field

The present disclosure relates to use of a heterocyclic compound for treating KRAS-associated cancers.

### Background

The RAS family is comprised of three members, KRAS, NRAS, and HRAS. KRAS is the single most frequently mutated oncogene in human cancers. KRAS mutations are prevalent in the cancerous cells of patients having any one of the three most refractory cancer types in the United States: 95% of pancreatic cancers, 45% of colorectal cancers, and 35% of lung cancers.

Because of the prevalence of KRAS mutations in particularly intractable cancers, intensive drug discovery efforts have been devoted to developing therapeutic strategies that block KRAS function. These efforts include (i) direct targeting approaches, such as disrupting protein-protein (e.g., RAS-Raf) interactions and covalent irreversible KRAS-G12C inhibition; and (ii) indirect targeting approaches, such as decreasing the RAS population at the plasma membrane and targeting downstream effector signaling proteins (e.g., ERK or mTOR). Despite extensive efforts, a clinically viable cancer therapy that effectively blocks KRAS function has remained elusive. Document WO 2014/176475 A2 discloses compounds that inhibit the interaction between HSP90 and proteins having an M-5 loop, such as EGFR, as well as their use in treating EGFR-related diseases, including cancers and solid tumors.

There is a need to develop new methods that effectively block KRAS function so as to treat KRAS-associated cancers.

### Summary

The present disclosure relates to treating KRAS-associated cancers with the heterocyclic compound, DGD1202, or a pharmaceutically acceptable salt thereof. Unexpectedly, DGD1202, as compared to known EGFR inhibitors, demonstrated superior antitumor efficacy against certain cancers specifically characterized by expression of mutant KRAS.

An aspect of the disclosure is a compound for use in treating a cancer characterized by expression of mutant KRAS protein and, optionally, mutated EGFR protein. This comprises administering to a subject in need thereof a therapeutic agent in an amount sufficient to alter KRAS-associated activity (e.g., KRAS or cMet) resulting from said KRAS mutation, wherein the therapeutic agent is a compound of Formula I (interchangeably referred to as "DGD1202"), shown below, or a pharmaceutically acceptable salt thereof:

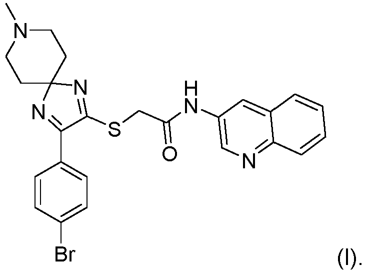

In some embodiments, the cancer is a KRAS-driven cancer. Examples of the KRAS-driven cancer include, but are not limited to, pancreatic cancer, colorectal cancer, head and neck cancer, and lung cancer.

In various embodiments, the cancer is characterized by presence of at least one deleterious KRAS mutation, which refers to a KRAS mutation, when present in a cancer cell, contributes to increased cellular proliferation. A deleterious KRAS mutation can be one of the following mutations: G12D, G12V, and G13D. The cancer may also be characterized by the presence of one or more of the following EGFR mutations: L858R, T790M, C797S, S768I, del Exon 19, or a combination thereof.

In an exemplary use, the KRAS mutation is G12D or G13D and the EGFR mutation is L858R or T790M.

In some embodiments, the cancer is resistant to an EGFR inhibitor (e.g., cetuximab or osimertinib).

In some embodiments, the above therapeutic agent is capable of degrading EGFR or blocking EGFR dimerization. Typically, the therapeutic agent is administered in an amount sufficient to alter the activity of KRAS. It can be administered (e.g., oral administration) in a dosage of 1 - 500 mg/kg (e.g., 10 - 100 mg/kg, 10 - 60 mg/kg, or 20 - 40 mg/kg).

Further disclosed are compositions comprising the therapeutic agent and a pharmaceutically acceptable carrier. The carrier in the pharmaceutical composition must be "acceptable" in the sense that it is compatible with the therapeutic agent of the composition (and preferably, capable of stabilizing the therapeutic agent) and not deleterious to the subject to be treated. One or more solubilizing agents can be utilized as pharmaceutical excipients for delivery of the therapeutic agent. Examples of other carriers include colloidal silicon oxide, magnesium stearate, cellulose, sodium lauryl sulfate, and D&C Yellow #10.

The above-described therapeutic agent can be administered to a subject orally, parenterally, by inhalation spray, topically, rectally, nasally, buccally, vaginally, or via an implanted reservoir. The term "parenteral" as used herein includes subcutaneous, intracutaneous, intravenous, intramuscular, intraarticular, intraarterial, intrasynovial, intrasternal, intrathecal, intralesional, and intracranial injection or infusion techniques.

A composition containing the above therapeutic agent for oral administration can be any orally acceptable dosage form including capsules, tablets, emulsions and aqueous suspensions, dispersions, and solutions. In the case of tablets, commonly used carriers include lactose and corn starch. Lubricating agents, such as magnesium stearate, are also typically added. For oral administration in a capsule form, useful diluents include lactose and dried corn starch. When aqueous suspensions or emulsions are administered orally, the therapeutic agent (i.e., DGD1202) can be suspended or dissolved in an oily phase combined with emulsifying or suspending agents. If desired, certain sweetening, flavoring, or coloring agents can be added. Oral solid dosage forms can be prepared by spray dried techniques; hot melt extrusion strategy, micronization, and nano milling technologies.

A nasal aerosol or inhalation composition can be prepared according to techniques well known in the art of pharmaceutical formulation. For example, such a composition can be prepared as a solution in saline, employing benzyl alcohol or other suitable preservatives, absorption promoters, fluorocarbons, and/or other solubilizing or dispersing agents known in the art. A composition having DGD1202 as the therapeutic agent can also be administered in the form of suppositories for rectal administration.

The term "treating" refers to application or administration of the therapeutic agent to a subject with the purpose to cure, alleviate, relieve, alter, remedy, improve, or affect the disease, the symptom, or the predisposition. "An effective amount" or "an amount effective" refers to the amount of the compound of Formula I which is required to confer the desired effect on the subject. Effective amounts vary, as recognized by those skilled in the art, depending on route of administration, excipient usage, and the possibility of co-usage with other therapeutic treatments such as use of other active agents.

### BRIEF DESCRIPTION OF THE FIGURES

Figure 1 depicts the effect of DGD1202 in mutant KRAS (and EGFR co-driven) head and neck (UMSCC74B) tumor model. KRAS driven UMSCC74B xenografts bearing nude mice were treated with vehicle, cetuximab (100 mg/kg, Monday), or DGD1202 (30 mg/kg, Mon-Fri) for 2 weeks and effect on tumor growth was measured and plotted.
Figure 2 depicts the effect of DGD1202 KRAS mutant cell-lines HCT116 and LoVo. Effect of DGD1202 (left line in each graph) in KRAS mutant colorectal cell lines (HCT116, Lovo) was assessed compared to cetuximab (right line in each graph) using a clonogenic assay.
Figure 3 depicts the effect of DGD1202 on EGFR, KRAS and downstream signaling. (A). HCT-116 (colorectal cancer) cells were treated with DGD1202 for 5h or 15h, and whole cell lysates were probed with listed antibodies. (B). Evaluation of DGD1202 effects against the KRAS G12D driven pancreatic cell line (Panc1) tumor model; effect of treatment on the steady-state level of EGFR and mtKRAS was determined by immunoblotting; and GAPDH expression was assessed as a loading control.
Figure 4 shows the effect of DGD1202 on pancreatic intraepithelial neoplasia (Panlns) in mutant KRAS mice compared to control.
Figure 5 depicts the effect of DGD1202 on osimertinib resistant, NCI-H1975 lung cancer xenografts. (A). SCID mice bearing NCI-H1975 osimertinib resistant xenografts were treated with DGD1202 (75 mg/kg, daily, PO). The arrow marks the initiation of treatment with DGD1202. Change in tumor volume is plotted. (B). The effect of DGD1202 treatment on EGFR was assessed in tumors harvested 3 days post DGD1202 treatment.
Figure 6 shows the percent lung lesion and total lung lesion of genetically engineered KRAS-LSL-G12D mice given DGD1202 compared to control.

### DETAILED DESCRIPTION

First disclosed in detail herein is a method of treating cancer characterized by expression of at least a KRAS mutation using a disclosed therapeutic agent.

KRAS plays a significant role in EGFR-induced signaling pathways. See, e.g, Knickelbein et al., Genes & Diseases, 2015, 2, 4-12 ("Knickelbein"). As reported in Knickelbein, activation of EGFR upon ligand binding and its subsequent auto-phosphorylation create a docking site for the SOS/GRB2 complex, resulting in nucleotide exchange by SOS and the GTP-bound form of KRAS; subsequently, KRAS signals through the RAF/MEK/ERK and PI3K/AKT cascades to promote cell growth and suppress apoptosis. As such, anti-EGFR antibodies, including cetuximab and panitumumab, bind to EGFR and prevent ligand binding and subsequent KRAS activation, leading to growth suppression and cell death due to the inhibition of the RAF/MEK/ERK and P13K/AKT pathways. See, Knickelbein, page 6, first paragraph. On the other hand, mutant KRAS can override the effect of anti-EGFR antibodies leading to cell growth and survival. See *Id.*

As pointed out above, this disclosure provides a method of treating a cancer that features at least a KRAS mutation.

More specifically, the method comprises administering to a subject in need thereof a therapeutic agent in an amount sufficient to alter KRAS-associated activity (e.g., KRAS or cMet) resulting from said KRAS mutation, wherein the therapeutic agent is a compound of Formula I, shown below, or a pharmaceutically acceptable salt thereof:

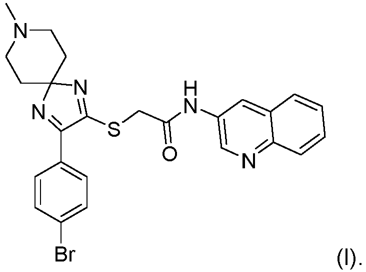

As set forth above, the cancer is characterized by presence of at least one deleterious KRAS mutation and, optionally, one or more EGFR mutations. The term "deleterious KRAS mutation" herein refers to a KRAS mutation, when present in a cancer cell, contributes to increased cellular proliferation. Examples of a deleterious KRAS mutation include, but are not limited to, G12D, G12V, and G13D. Examples of an EGFR mutation include, but are not limited to, L858R, T790M, C797S, S768I, and del Exon 19. In one embodiment, the deleterious KRAS mutation is G12D or G13D, and the EGFR mutation is L858R or T790M.

The above described compound shows significant improvement, as compared to structurally close analogs, in pharmacological properties as well as biological activities. For example, DGD1202 demonstrates much better liver microsomal stability as compared to two other structurally close compounds shown below:

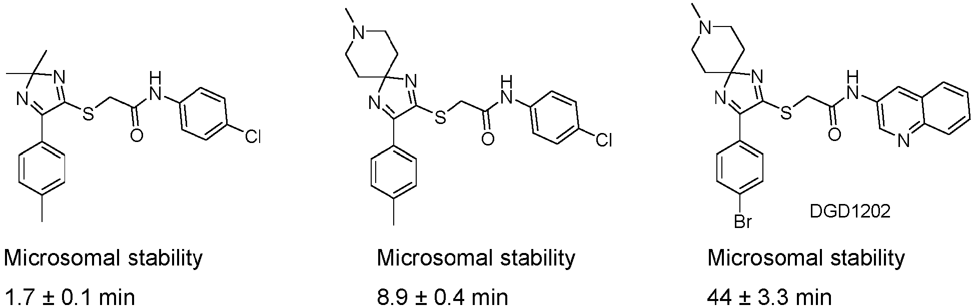

DGD1202 is stable with a liver microsomal half-life over 46 minutes, soluble in water at pH 3.5, bioavailable via both systemic injection and oral administration, and potent with a sub-micromolar IC₅₀ in the clonogenic cellular assay. Importantly, this compound degrades EGFR instead of inhibiting the activity thereof. It also blocks EGF-induced EGFR dimerization, and directly binds to purified EGFR, and is selectively active in EGFR-driven osimertinib resistant cell lines and in xenograft models.

To further explore the antitumor activity of the compound of Formula I (i.e., DGD1202), a NCI-60 cell line screen was performed. The data of this experiment (see the table in Example 3 below) suggests that this compound is active not only against tumor cells that are driven by EGFR but also against a variety of cells that express mutant KRAS and show resistance to an EGFR targeting antibody, as cetuximab.

The IC₅₀ value of DGD1202 against these cetuximab-resistant cell-lines ranges from 0.5 µM to 2.2 µM. In immortalized cells of non-cancer origin, the IC₅₀ value of this compound is well over 20 µM.

In addition, DGD1202 shows activities against mutant KRAS positive cell lines as well as in a transgenic mouse model where KRAS G12D is expressed in the pancreas that causes the formation of pancreatic intraepithelial neoplasia (Panlns). Moreover, it further shows activity in a cetuximab-resistant head and neck tumor model where KRAS is mutated (UMSCC74B).

Cetuximab (Erbitux, anti-EGFR antibody) has demonstrated a benefit in colorectal cancer (CRC) patients. Yet, only subsets of patients with WT-EGFR have shown a durable clinical response. Patients with KRAS mutations typically do not respond to inhibition of EGFR kinase activity with cetuximab treatment. Recent studies from various groups using preclinical models have shown that degradation of the oncoprotein is more effective than inhibition of the kinase activity. See, e.g., Raina et al., Proc Natl Acad Sci USA., 2016, 113:7124-7129; and Corcoran et al., Cancer Discovery, 2012, 2:227-235. This could be because the EGFR protein-scaffold might continue to function by interacting with other proteins even upon inhibition of its kinase activity. An important advantage of degrading an oncoprotein (over inhibition of its activity) is that conceivably the rate of acquired mutation-mediated-resistance is expected to be lower. In addition to acquired mutations, even the patients who initially respond to EGFR inhibitor therapy, will become resistant due to upregulation of compensatory signaling. In CRC, the EGFR-KRAS axis is known to activate MEK-ERK signaling, which promotes cancer progression, causing both primary and secondary resistance to existing kinase inhibitor therapies. In KRAS mutant CRC tumors, EGFR continues to signal via ERK pathway, even when the ERK pathway is inhibited, thereby causing resistance to EGFR inhibitor therapy. Therefore, the method of using the compound of Formula I for treating cancer featuring at least a KRAS mutation provides unexpected superiority over known treatment.

To practice the above method of treatment, a pharmaceutically acceptable salt of the compound of Formula I can be used. As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge et al. describe pharmaceutically acceptable salts in detail in J. Pharmaceutical Sciences, 1977, 66, 1-19. Pharmaceutically acceptable salts of the compound above include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, trifluoroacetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, glutamate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Salts of compounds containing a carboxylic acid or other acidic functional group can be prepared by reacting with a suitable base. Such salts include, but are not limited to, alkali metal, alkaline earth metal, aluminum salts, ammonium, N⁺(C₁₋₄alkyl)₄ salts, and salts of organic bases such as trimethylamine, triethylamine, morpholine, pyridine, piperidine, picoline, dicyclohexylamine, N,N'-dibenzylethylenediamine, 2-hydroxyethylamine, bis-(2-hydroxyethyl)amine, tri-(2-hydroxyethyl)amine, procaine, dibenzylpiperidine, dehydroabietylamine, N,N'-bisdehydroabietylamine, glucamine, N-methylglucamine, collidine, quinine, quinoline, and basic amino acids such as lysine and arginine. This disclosure also envisions the quaternization of any basic nitrogen-containing groups of the compound provided herein. Water or oil-soluble or dispersible products may be obtained by such quaternization. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate and aryl sulfonate. Exemplary pharmaceutically acceptable salts of the compound of formula I include, but are not limited to, hydrochloride, hydrobromide, sulfate, nitrate, phosphate, mesylate, esylate, isethionate, tosylate, napsylate, besylate, acetate, propionate, maleate, benzoate, salicylate, fumerate, glutamate, aspartate, citrate, lactate, succinate, tartrate, glycollate, hexanoate, octanoate, decanoate, oleate, stearate, pamoate, and polystryrene sulfonate.

In some embodiments not within the claimed scope, a prodrug of the compound of Formula I (DGD1202) can also be used. As used herein, the term "prodrug" refers to a medication or compound that, after administration, is metabolized (i.e., converted within the body) into a pharmacologically active drug. Thus, a prodrug of DGD1202 refers to a medication or compound that, after administration, is converted within the body into DGD1202. To a skilled artisan, a corresponding prodrug can be used instead to improve how a medicine is absorbed, distributed, metabolized, and excreted, thereby improving the pharmacological effect exerted in a subject (e.g., a human) in need thereof. Examples of a prodrug that can be used in this invention include, but are not limited to, amides, carbamates, sulfonamides, carboxamides, N-oxides, N-acyloxyalkyl derivatives, N-hydroxyalkyl derivatives, and N-(phosphoryloxy)alkyl derivatives.

Provided below is a general synthesis of the compound of Formula I:

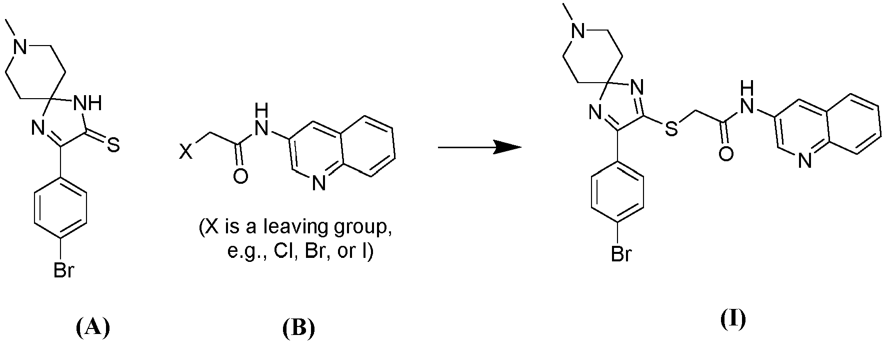

Typically, acetamide B (1 equiv.) is added to a solution of 3-(4-bromophenyl)-8-methyl-1,4,8-triazaspiro[4.5]dec-3-ene-2-thione A in an organic solvent (e.g., anhydrous acetonitrile). The reaction mixture is warmed to a pre-determined temperature (e.g., 40 °C). Next, a base (e.g., 2M aqueous potassium carbonate solution, 1 equiv.) is added to the reaction mixture. The reaction is maintained at the same temperature until TLC shows loss of starting materials and emergence of a new R_{f} spot (typically 2-6 hours). Once the reaction is complete by TLC, it is worked up and the resulting crude compound is purified by flash chromatography to afford the desired product: (2-((3-(4-bromophenyl)-8-methyl-1,4,8-triazaspiro[4.5]deca-1,3-dien-2-yl)thio)-N-(quinolin-3-yl)acetamide).

Methods for synthesizing intermediates A and B for preparing the compound of Formula I are well known in the art. See, for example, R. Larock, Comprehensive Organic Transformations (2nd Ed., VCH Publishers 1999); P. G. M. Wuts and T. W. Greene, Greene's Protective Groups in Organic Synthesis (4th Ed., John Wiley and Sons 2007); L. Fieser and M. Fieser, Fieser and Fieser's Reagents for Organic Synthesis (John Wiley and Sons 1994); L. Paquette, ed., Encyclopedia of Reagents for Organic Synthesis (2nd ed., John Wiley and Sons 2009); P. Roszkowski, J.K. Maurin, Z. Czarnocki "Enantioselective synthesis of (R)-(-)-praziquantel (PZQ)" Tetrahedron: Asymmetry 17 (2006) 1415-1419; and L. Hu, S. Magesh, L. Chen, T. Lewis, B. Munoz, L. Wang "Direct inhibitors of keap1-nrf2 interaction as antioxidant inflammation modulators," WO2013/067036.

The compound of Formula I or its pharmaceutically acceptable salt described herein, termed as the therapeutic agent, can be administered to a subject in a therapeutically effective amount (e.g., in an amount sufficient to prevent or relieve the symptoms of a disease or disorder associated with a KRAS mutation). The therapeutic agent can be administered alone or as part of a pharmaceutically acceptable composition. The therapeutic agent can be administered all at once, multiple times, or delivered substantially uniformly over a period of time.

A skilled artisan will appreciate that the dosage of the therapeutic agent can be varied over time. A particular administration regimen for a particular subject will depend, in part, upon the compound, the amount of compound administered, the route of administration, and the cause and extent of any side effects. The amount of compound administered to a subject (e.g., a mammal, such as a human) in accordance with the disclosure should be sufficient to effect the desired response over a reasonable time frame. Dosage typically depends upon the route, timing, and frequency of administration. Accordingly, the clinician titers the dosage and modifies the route of administration to obtain the optimal therapeutic effect, and conventional range-finding techniques are known to those of ordinary skill in the art.

Purely by way of illustration, the above-described method of treating cancer comprises administering, e.g., from about 1 mg/kg up to about 500 mg/kg of the compound of Formula I, depending on the factors mentioned above. In other embodiments, the dosage ranges can be from 5 mg/kg up to about 100 mg/kg; or 10 mg/kg up to about 100 mg/kg, or 10 mg/kg up to about 60 mg/kg, or 20 mg/kg up to about 40 mg/kg. Some conditions require prolonged treatment, which may or may not entail administering lower doses of compound over multiple administrations. If desired, a dosage of the compound is administered as two, three, four, five, six or more sub-doses administered separately at appropriate intervals throughout the day, optionally, in unit dosage forms. The treatment period will depend on the particular condition, and may last one day to several months.

Suitable methods of administering a pharmaceutically acceptable composition comprising the compound of Formula I are well known in the art. Although more than one route can be used to administer a compound, a particular route can provide a more immediate and more effective reaction than another route. Depending on the circumstances, a pharmaceutical composition comprising the compound is applied or instilled into body cavities, absorbed through the skin or mucous membranes, ingested, inhaled, and/or introduced into circulation. For example, in certain circumstances, it will be desirable to deliver a pharmaceutical composition comprising the therapeutic agent orally, through injection, or by one of the following means: intravenous, intraperitoneal, intracerebral (intra-parenchymal), intracerebroventricular, intramuscular, intra-ocular, intraarterial, intraportal, intralesional, intramedullary, intrathecal, intraventricular, transdermal, subcutaneous, intraperitoneal, intranasal, enteral, topical, sublingual, urethral, vaginal, or rectal. The compound can be administered by sustained release systems, or by implantation devices.

To facilitate administration, the therapeutic agent is, in various aspects, formulated into a pharmaceutically acceptable composition comprising a carrier (e.g., vehicle, adjuvant, or diluent). The particular carrier employed is limited only by chemico-physical considerations, such as solubility and lack of reactivity with the compound, and by the route of administration. Pharmaceutically acceptable carriers are well known in the art. Illustrative pharmaceutical forms suitable for injectable use include sterile aqueous solutions or dispersions and sterile powders for the extemporaneous preparation of sterile injectable solutions or dispersions (for example, see U.S. Patent No. 5,466,468). Injectable compositions are further described in, e.g., Pharmaceutics and Pharmacy Practice, J. B. Lippincott Co., Philadelphia. Pa., Banker and Chalmers, eds., pages 238-250 (1982), and ASHP Handbook on Injectable Drugs, Toissel, 4th ed., pages 622-630 (1986)). A pharmaceutical composition comprising the therapeutic agent is, in one aspect, placed within containers, along with packaging material that provides instructions regarding the use of such pharmaceutical compositions. Generally, such instructions include a tangible expression describing the reagent concentration, as well as, in certain embodiments, relative amounts of excipient ingredients or diluents (e.g., water, saline or PBS) that may be necessary to reconstitute the pharmaceutical composition.

Compositions suitable for parenteral injection may comprise physiologically acceptable sterile aqueous or nonaqueous solutions, dispersions, suspensions, or emulsions, and sterile powders for reconstitution into sterile injectable solutions or dispersions. Examples of suitable aqueous and nonaqueous carriers, diluents, solvents, or vehicles include water, ethanol, polyols (propylene glycol, polyethylene glycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions, and by the use of surfactants.

These compositions may also contain adjuvants such as preserving, wetting, emulsifying, and dispersing agents. Microorganism contamination can be prevented by adding various antibacterial and antifungal agents, for example, parabens, chlorobutanol, phenol, sorbic acid, and the like. It may also be desirable to include isotonic agents, for example, sugars, sodium chloride, and the like. Prolonged absorption of injectable pharmaceutical compositions can be brought about by the use of agents delaying absorption, for example, aluminum monostearate and gelatin.

Solid dosage forms for oral administration include capsules, tablets, powders, and granules. In such solid dosage forms, the therapeutic agent is admixed with at least one inert customary excipient (or carrier) such as sodium citrate or dicalcium phosphate or (a) fillers or extenders, as for example, starches, lactose, sucrose, mannitol, and silicic acid; (b) binders, as for example, carboxymethylcellulose, alginates, gelatin, polyvinylpyrrolidone, sucrose, and acacia; (c) humectants, as for example, glycerol; (d) disintegrating agents, as for example, agar-agar, calcium carbonate, potato or tapioca starch, alginic acid, certain complex silicates, and sodium carbonate; (e) solution retarders, as for example, paraffin; (f) absorption accelerators, as for example, quaternary ammonium compounds; (g) wetting agents, as for example, cetyl alcohol and glycerol monostearate; (h) adsorbents, as for example, kaolin and bentonite; and (i) lubricants, as for example, talc, calcium stearate, magnesium stearate, solid polyethylene glycols, sodium lauryl sulfate, or mixtures thereof. In the case of capsules, and tablets, the dosage forms may also comprise buffering agents. Solid compositions of a similar type may also be used as fillers in soft and hard filled gelatin capsules using such excipients as lactose or milk sugar, as well as high molecular weight polyethylene glycols, and the like.

Solid dosage forms such as tablets, dragees, capsules, pills, and granules can be prepared with coatings and shells, such as enteric coatings and others well known in the art. The solid dosage forms may also contain opacifying agents. Further, the solid dosage forms may be embedding compositions, such that they release the therapeutic agent in a certain part of the intestinal tract in a delayed manner. Examples of embedding compositions that can be used are polymeric substances and waxes. The compound of Formula I can also be in micro-encapsulated form, optionally with one or more excipients.

Liquid dosage forms for oral administration include pharmaceutically acceptable emulsions, solutions, suspensions, syrups, and elixirs. In addition to the therapeutic agent, the liquid dosage form may contain inert diluents commonly used in the art, such as water or other solvents, solubilizing agents and emulsifiers, as for example, ethyl alcohol, isopropyl alcohol, ethyl carbonate, ethyl acetate, benzyl alcohol, benzyl benzoate, propylene glycol, 1,3-butylene glycol, dimethylformamide, oils, in particular, cottonseed oil, groundnut oil, corn germ oil, olive oil, castor oil, and sesame seed oil, glycerol, tetrahydrofurfuryl alcohol, polyethylene glycols and fatty acid esters of sorbitan, or mixtures of these substances, and the like.

Besides such inert diluents, the composition can also include adjuvants, such as wetting agents, emulsifying and suspending agents, sweetening, flavoring, and perfuming agents. Suspensions, in addition to the therapeutic agent, may contain suspending agents, as for example, ethoxylated isostearyl alcohols, polyoxyethylene sorbitol and sorbitan esters, microcrystalline cellulose, aluminum metahydroxide, bentonite, agar-agar, and tragacanth, or mixtures of these substances, and the like.

Upon formulation, solutions can be administered in a manner compatible with the dosage formulation and in such amount as is therapeutically effective. The compositions are easily administered in a variety of dosage forms such as injectable solutions, drug release capsules and the like. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration.

The compound of Formula I can modulate EGFR in a unique way. In some embodiments, the compound blocks or inhibits EGFR dimerization. In various embodiments, the compound induces EGFR degradation.

Although EGFR has been identified as an oncogene and an important molecular target in cancer, there is still a great need and opportunity for an improved approach to modulate the activity of this oncogene. Using a cell penetrating peptide that blocks dimerization (Disruptin) or siRNA, it has been shown that EGFR degradation has a profound effect on cell survival, even in TKI resistant cells. See, e.g., Raina et al., Proc Natl Acad Sci USA., 2016, 113:7124-7129. By degrading the EGFR protein rather than simply inhibiting its kinase activity, a broad spectrum of activities has been demonstrated in preclinical models while improving the ability to target tumor tissue due to the fact that an agent affects only EGF-bound EGFR, which is abundant in tumor cells compared to normal tissue, thereby, improving the safety profile and the therapeutic window.

The approach of degrading EGFR rather than simply inhibiting its kinase activity overcomes the resistance to osimertinib that invariably develops in patients with non-small cell lung cancer. See, e.g., Corcoran et al., Cancer Discovery, 2012, 2:227-235. While the focus of the application of EGFR degradation is on lung cancers, additional and important clinical opportunities also exist in other cancers that are driven by EGFR or KRAS, such as head & neck cancer, colorectal cancer, and pancreatic cancer.

Any reference to methods of treatment of the human (or animal) body by therapy in this description is to be construed as reference to the compounds, pharmaceutical compositions and medicaments of the present invention for use in such a method of treatment. As used herein, the term "treat," as well as words related thereto, do not necessarily imply 100% or complete treatment. Rather, there are varying degrees of treatment of which one of ordinary skill in the art recognizes as having a potential benefit or therapeutic effect. In this respect, the methods of treating cancer of the present disclosure can provide any amount or any level of treatment of cancer. Furthermore, the treatment provided by the methods of the present disclosure may include treatment of one or more conditions or symptoms of the cancer, being treated. Also, the treatment provided by the methods of the present disclosure may encompass slowing the progression of the cancer. For example, the disclosed methods can treat cancer by virtue of reducing tumor or cancer growth, reducing metastasis of tumor cells, increasing cell death of tumor or cancer cells, and the like.

The cancer treatable by the methods disclosed herein may be any KRAS-associated cancer or KRAS-driven cancer, which is characterized by at least a KRAS mutation.

The cancer in some aspects is one selected from the group consisting of pancreatic cancer, colorectal cancer, head and neck cancer, lung cancer, e.g., non-small cell lung cancer (NSCLC), ovarian cancer, cervical cancer, gastric cancer, breast cancer, hepatocellular carcinoma, glioblastoma, liver cancer, malignant mesothelioma, melanoma, multiple myeloma, prostate cancer, and renal cancer. In some embodiments, the cancer is pancreatic cancer, colorectal cancer, head and neck cancer, or lung cancer. In some embodiments, the cancer is cetuximab-resistant cancer or osimertinib-resistant cancer.

Disclosed, but not within the claimed scope, is a method of altering the level of KRAS or cMet in a cell characterized by expression of at least a KRAS mutation. This method comprises contacting said cell with the compound of Formula I in an amount effective to degrade the activity of EGFR in said cell.

Typically, the KRAS- or cMet-associated cell is characterized by presence of at least one KRAS mutation and, optionally, one or more EGFR mutations. Examples of a KRAS mutation include, but are not limited to, G12D, G12V, and G13D. Examples of an EGFR mutation include, but are not limited to, L858R, T790M, C797S, S768I, and del Exon 19. In some embodiments, the KRAS mutation is G12D or G13D, and the EGFR mutation is L858R or T790M.

In some embodiments, the disclosed methods of administering the compound of Formula I results in either degradation EGFR or block of EGFR dimerization, thereby altering the level of KRAS or cMet in the cell.

Without further elaboration, it is believed that one skilled in the art can, based on the above description, utilize the present disclosure to its fullest extent. The following specific examples, i.e., EXAMPLES 1-5, are therefore to be construed as merely illustrative, and not limitative of the remainder of the disclosure in any way whatsoever.

### EXAMPLES

### Example 1 - Synthesis and Characterization of DGD1202

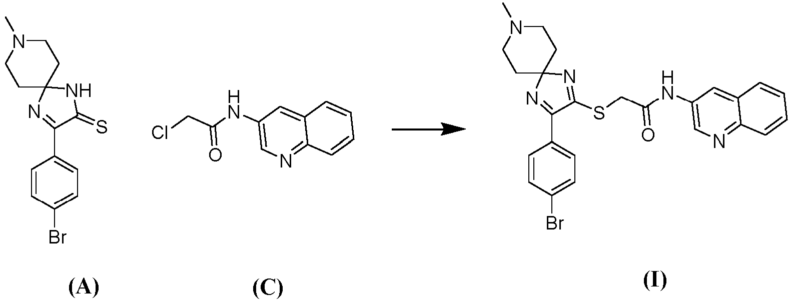

To a solution of 3-(4-bromophenyl)-8-methyl-1,4,8-triazaspiro[4.5]dec-3-ene-2-thione A in anhydrous acetonitrile was added acetamide C (1 equiv.). The reaction mixture was warmed to 40 °C. Next, 2M aqueous potassium carbonate solution (1 equiv.) was added to the reaction mixture. The reaction was maintained at 40 °C until TLC showed loss of starting materials and emergence of a new R_{f} spot (typically 2-6 hours). Once the reaction was complete by TLC, it was worked up. The crude reaction mixture was poured into a separatory funnel, and ethyl acetate and water were added. The organic layer was separated and then washed with brine (1 x). The organic layer was then dried over anhydrous MgSO₄, filtered, and the filtrate was concentrated under reduced pressure afford the crude product. The crude product was purified by flash chromatography with elution occurring in 10% methanol/dichloromethane solution to afford DGD1202: (2-((3-(4-bromophenyl)-8-methyl-1,4,8-triazaspiro[4.5]deca-1,3-dien-2-yl)thio)-N-(quinolin-3-yl)acetamide). ¹H NMR (400MHz, DMSO-d₆) δ 10.89 (br s, 1H), 8.95 (s, 1H), 8.67 (s, 1H), 7.96 (br d, J=8.33 Hz, 1H), 7.92 (br d, J=8.33 Hz, 1H), 7.78-7.87 (m, 4H), 7.52-7.72 (m, 2H), 4.29 (s, 2H), 2.43-2.52 (m, 4 H), 2.18 (br s, 3H), 1.55-1.85 (m, 4H); MS (ESI + m/z 523.05, ESI- m/z 521.00 ); TLC: (90:10:0.5, DCM:MeOH:NH₄OH) R_{f} =0.47.

### Example 2 - Evaluation of DGD1202 in KRAS Mutant Head and Neck Cancer

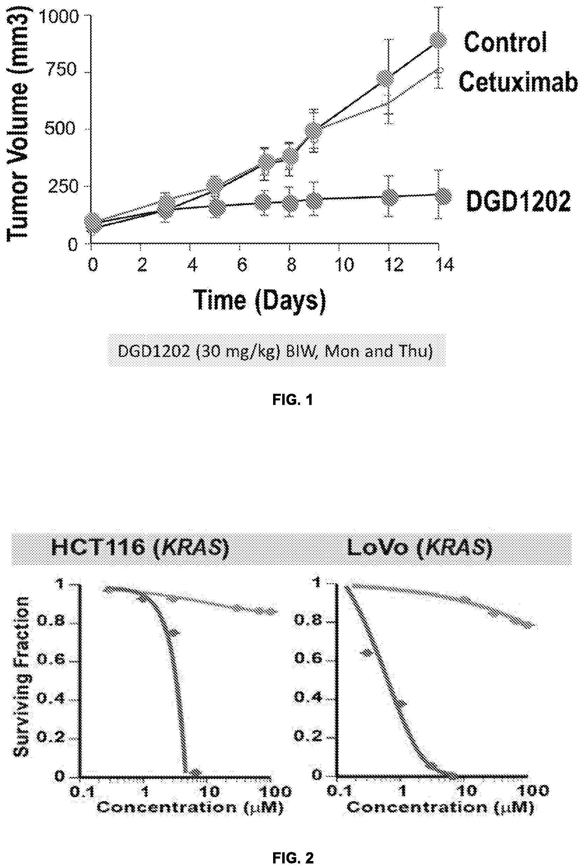

A study was conducted to evaluate the efficacy and toxicity of DGD1202 in KRAS G12D driven, cetuximab-resistant tumor model of head and neck cancer (UMSCC74B) (Figure 1).

Tumor-bearing mice were treated with DGD1202 via oral gavage in 30 mg/kg dose biweekly for one week. This dose was selected based on its single-dose PK profile. The resulting effect of DGD1202 on tumor volume was compared to control mice which did not receive the test compound, and control mice which received cetuximab, a known EGFR inhibitor.

Unexpectedly, the dose and regimen in this treatment was safe and mice treated with DGD1202 showed significant tumor growth suppression (*P* <*0.001*) and, by sharp contrast, cetuximab failed to exhibit meaningful efficacy.

### Example 3- Evaluation of DGD1202 in KRAS Mutant Colorectal and Pancreatic Cancer Cell Lines

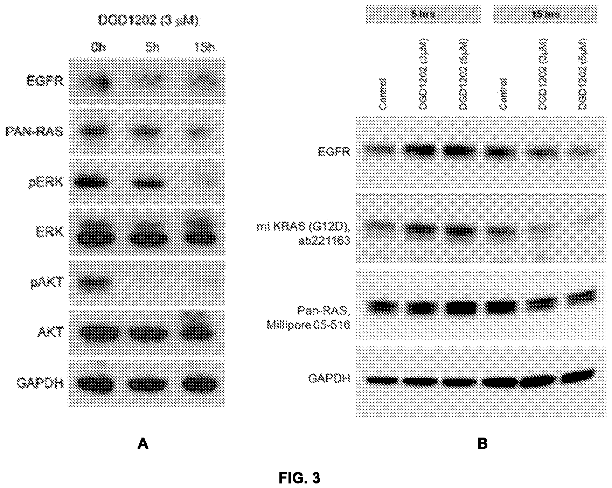

A study was conducted to evaluate the activity of DGD1202 in a KRAS G13D driven cetuximab-resistant colorectal cell-line (HCT-116) using clonogenic survival assays (Figure 2) and in a pancreatic cancer cell line (Panc1) that contains KRAS G12D mutation (Figure 3B).

More specifically, cells were plated at clonal density in 60 or 100 mm culture dishes in triplicate one day before treatment with a range of concentrations (e.g., 0 - 10 micro M). Eight to twelve days later, cells were fixed with acetic acid/methanol (1:7, v/v), stained with crystal violet (0.5%, w/v), and counted using a stereomicroscope. Drug cytotoxicity (surviving drug-treated cells) was measured and normalized to the survival of the untreated control cells.

The data shown in Figures 2 and 3B suggest that DGD1202 was effective in treating mutant KRAS driven colorectal and pancreatic cancer cells. It is hypothesized that antitumor efficacy is due to a loss of EGFR protein that blocks reactivation of ERK/AKT signaling and is likely to occur via EGFR.

The effect of DGD1202 on EGFR, ERK and AKT was also evaluated by immunoblotting. The preliminary data for DGD1202 in mutant KRAS driven HCT-116 (CRC) and Panc1 (Pancreas) cell lines are shown in Figure 3A and Figure 3B, respectively. It was observed that this compound affected both EGFR and mtKRAS (Panc1) levels as detected by KRAS G12D specific antibody in case of Panc1 cell line and by PAN-RAS antibody in case of HCT116, which in turn affected downstream signaling in pAKT and pERK.

The immunoblotting was performed by following the protocol below:

Cells were plated in 60-mm dishes at a density of 3×10⁵ cells per dishes and incubated overnight or to 70 % confluence. The cells were treated with the vehicle (DMSO) or DGD1202 and then were harvested at various time points. The pellets were washed twice with ice-cold PBS and re-suspended in lysis buffer for 30 min. After sonication, particulate material was removed by centrifugation at 13,000 rpm for 10 min at 4 °C. The soluble protein fraction was heated to 95 °C for 5 min and then applied to a 4-12% Bis-Tris precast gel (Invitrogen) and transferred onto a PVDF membrane. Membranes were incubated for 1 hour at room temperature in blocking buffer consisting of 5% BSA and 1% normal goat serum in Tris-buffered saline (137 mM NaCl, 20 mM Tris-HCl (pH 7.6), 0.1% (v/v) Tween 20). Membranes were subsequently incubated overnight at 4 °C with the primary antibody in blocking buffer, washed, and incubated for 1 hour with horseradish peroxidase-conjugated secondary antibody. After three additional washes in Tris-buffered saline, bound antibody was detected by enhanced chemiluminescence plus reagent. For quantification of relative protein levels, immunoblot films were scanned and analyzed using Image J 1.32j software. The relative protein levels shown represent a comparison with untreated controls.

In this connection, the activity of DGD1202 was also tested against 60 different human tumor cell lines at the National Cancer Institute, using the standard NCI 60 screening protocol. The percent growth inhibition for the top performing cell lines is given in the table below. The top responding cells lines (e.g., HCT-116) show mutations in KRAS genes (e.g., KRAS G13D), which are frequently mutated in CRC and pancreas and correlate with resistance to cetuximab.

| Panel | Cell Line | Percent Growth | BRAF | KRAS |
|---|---|---|---|---|
| Melanoma | SK-MEL-5 | -96.2 | BRAF V600E | |
| Colon Cancer | HCT-116 | -90.7 | | KRAS G13D |
| Melanoma | M14 | -83.8 | BRAF V600E | |
| Renal Cancer | 786-0 | -81.7 | | |
| Melanoma | UACC-62 | -78.9 | BRAF V600E | |
| Melanoma | LOX IMVI | -78.5 | BRAF V600E | |
| Colon Cancer | COLO 205 | -76.2 | BRAF V600E | |
| Melanoma | MALME-3M | -75.5 | BRAF V600E | |
| Melanoma | SK-MEL-28 | -70.1 | BRAF V600E | |
| Colon Cancer | HT29 | -67.1 | BRAF V600E | |
| Leukemia | K-562 | -61.5 | | |
| Melanoma | UACC-257 | -61.2 | BRAF V600E | |
| Colon Cancer | HCC-2998 | -51.3 | | |
| Breast Cancer | MDA-MB-468 | -43.8 | | |
| Breast Cancer | MCF7 | -42.7 | | |
| Leukemia | HL-60(TB) | -40.7 | | NRAS p.Q61L |
| Breast Cancer | MDA-MB-231/ATCC | -40.5 | BRAF G464V | KRAS G13D |

### Example 4 - Evaluation of DGD1202 in KRAS Mutant Pancreatic Cancer

A study was conducted to evaluate the efficacy of DGD1202 in KRAS G12D driven pancreatic tumor model.

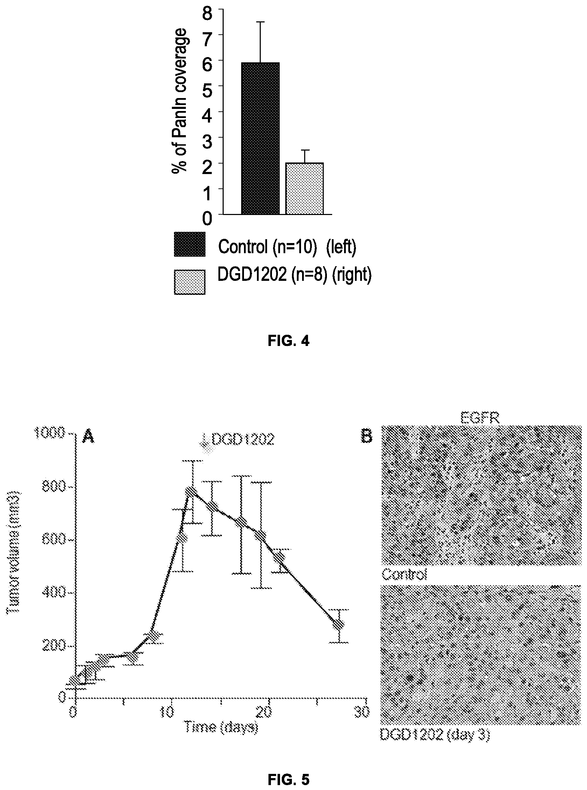

More specifically, 6-week old KC mice were treated with DGD1202 via oral gavage (30 mg/kg body weight, daily) for 5 weeks. The resulting effect on Panln (pancreatic intraepithelial neoplasia) levels were observed compared to control mice which did not receive DGD1202. At week 11 all the mice were sacrificed and effect on Panln was scored, as shown in Figure 4. The mice treated with DGD1202 showed significantly reduced propensity for developing Panln, a type of pancreatic duct lesion.

These data indicate that DGD1202 unexpectedly exhibited efficacy in treating mutant KRAS driven pancreatic cancer.

### Example 5 - Evaluation of DGD1202 in EGFR Mutant Lung Cancer

A study was conducted to evaluate the efficacy of DGD1202 in mutant EGFR driven lung cancer model (Figure 5).

Briefly, SCID mice bearing locally advanced NCI-H1975-AZR (osimertinib resistant) xenograft were treated daily with oral gavage of DGD1202 (75 mg/kg) for two weeks. Tumor volumes were measured using calipers at least three times a week. The effect of DGD1202 treatment on EGFR expression was determined by IHC staining 3-days after initiation of treatment.

The data shown in Figure 5 indicate that DGD1202 unexpectedly exhibited efficacy in treating mutant EGFR driven lung cancer, which is resistant to osimertinib.

### Example 6 - Evaluation of DGD1202 in Genetically Engineered KRAS-LSL-G12D Mouse Model for Lung Cancer

The efficacy of DGD1202 treatment was assessed for KRAS-mutant lung tumor initiation and progression. A genetically engineered KRAS-LSL-G12D mouse model of lung cancer was used. This mouse carries a Lox-Stop-Lox (LSL) sequence followed by the KRAS G12D point mutation allele. Tumorigenesis was initiated by intra-nasal delivery of Adenovirus-Cre particles (1.5x10e⁷ pfu) that transduced lung epithelial cells to express Cre recombinase that deletes the LSL cassette and allows the expression of the mutant KRAS oncogenic protein. These mice showed hyperplasia of the lung by 6-8 weeks and adenomas by 12 weeks of age.

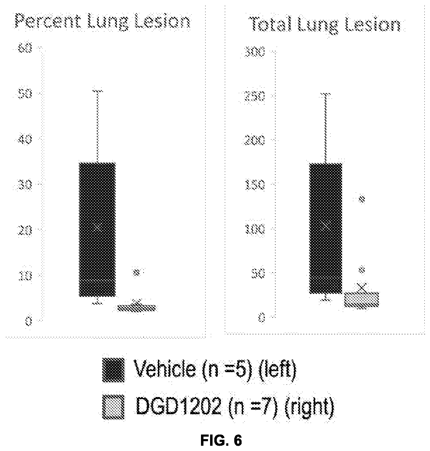

Eight weeks after viral delivery, the mice were treated either with vehicle or with DGD1202 on a Monday-Friday schedule for 4 weeks at a dose of 30 mg/kg oral gavage. When mice were 20-week old, the lungs were harvested and the H&E sections underwent morphological assessment. The histopathological spectrum of the lung lesions was noted and recorded in a double-blinded fashion for hyperplasia- alveolar or bronchial, the pattern of hyperplasia, adenoma, hyperplasia with focal atypia, bronchial dysplasia, adenomas, and atypical adenomatous hyperplasia. The percentage of lung lesion and total area of lung lesion from each lobe in every sample from both treatment group were scored and are shown in Fig. 6. The paired t-test shows that the difference between the 2 cohorts tend towards significance (p=0.0577).

### OTHER EMBODIMENTS

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

Further, from the above description, one skilled in the art can easily ascertain the essential characteristics of the present disclosure, and without departing from the scope thereof, can make various changes and modifications of the disclosure to adapt it to various usages and conditions. Thus, other embodiments are also within the claims.

## Claims

1. A compound of Formula I, or a pharmaceutically acceptable salt thereof: 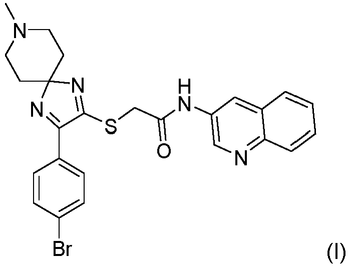 for use in the treatment of a cancer **characterized by** expression of at least a KRAS mutation, wherein the compound of Formula I alters the activity of KRAS or cMet resulting from said KRAS mutation.

2. The compound or salt for use of claim 1, wherein the cancer is a KRAS-driven cancer.

3. The compound or salt for use of claim 2, wherein the cancer is selected from the group consisting of pancreatic cancer, colorectal cancer, head and neck cancer, and lung cancer.

4. The compound or salt for use of any one of claims 1 to 3, wherein the cancer is **characterized by** expression of at least one KRAS mutation selected from the group consisting of G12D, G12V, and G13D; and, optionally, an EGFR mutation selected from the group consisting of L858R, T790M, C797S, S768I, del Exon 19, and a combination thereof, preferably wherein the KRAS mutation is G12D or G13D.

5. The compound or salt for use of claim 4, wherein the EGFR mutation is L858R or T790M.

6. The compound or salt for use of any one of claims 1 to 5, wherein the cancer is resistant to an EGFR inhibitor.

7. The compound or salt for use of claim 6, wherein the EGFR inhibitor is cetuximab or osimertinib.

8. The compound or salt for use of any one of claims 1 to 7, formulated for administration in a dosage of 1 - 500 mg/kg.

9. The compound or salt for use of any one of claims 1 to 8, formulated for oral administration.

10. The compound or salt for use of claim 1, wherein the cancer is resistant to an EGFR inhibitor, and is **characterized by** expression of at least one KRAS mutation selected from the group consisting of G12D, G12V, and G13D; and, optionally, an EGFR mutation selected from the group consisting of L858R, T790M, C797S, and a combination thereof.

11. The compound or salt for use of claim 10, wherein the cancer is a KRAS-driven cancer and the compound or salt is orally administered in a dosage of 1 - 500 mg/kg.

12. The compound or salt for use of claim 11, wherein the cancer is selected from the group consisting of pancreatic cancer, colorectal cancer, head and neck cancer, and lung cancer and the compound or salt is orally administered in a dosage of 20 - 40 mg/kg.

## Patentansprüche

1. Verbindung der Formel I oder pharmazeutisch verträgliches Salz davon: 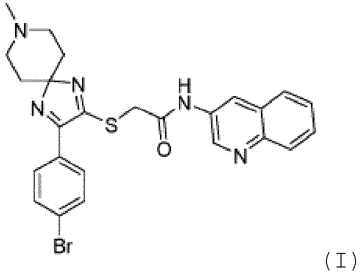 zur Verwendung bei der Behandlung einer Krebserkrankung, die **gekennzeichnet ist durch** die Expression von mindestens einer KRAS-Mutation, wobei die Verbindung der Formel I die Aktivität von KRAS oder cMet, die aus der KRAS-Mutation resultiert, verändert.

2. Verbindung oder Salz zur Verwendung nach Anspruch 1, wobei die Krebserkrankung eine durch KRAS ausgelöste Krebserkrankung ist.

3. Verbindung oder Salz zur Verwendung nach Anspruch 2, wobei die Krebserkrankung ausgewählt ist aus der Gruppe bestehend aus Pankreaskrebs, Dickdarmkrebs, Kopf- und Halskrebs und Lungenkrebs.

4. Verbindung oder Salz zur Verwendung nach einem der Ansprüche 1 bis 3, wobei die Krebserkrankung **gekennzeichnet ist durch** die Expression von mindestens einer KRAS-Mutation, die ausgewählt ist aus der Gruppe bestehend aus G12D, G12V und G13D; und gegebenenfalls einer EGFR-Mutation, die ausgewählt ist aus der Gruppe bestehend aus L858R, T790M, C797S, S768I, del Exon 19 und einer Kombination davon, wobei die KRAS-Mutation vorzugsweise G12D oder G13D ist.

5. Verbindung oder Salz zur Verwendung nach Anspruch 4, wobei die EGFR-Mutation L858R oder T790M ist.

6. Verbindung oder Salz zur Verwendung nach einem der Ansprüche 1 bis 5, wobei die Krebserkrankung gegen einen EGFR-Inhibitor resistent ist.

7. Verbindung oder Salz zur Verwendung nach Anspruch 6, wobei der EGFR-Inhibitor Cetuximab oder Osimertinib ist.

8. Verbindung oder Salz zur Verwendung nach einem der Ansprüche 1 bis 7, formuliert zur Verabreichung in einer Dosierung von 1-500 mg/kg.

9. Verbindung oder Salz zur Verwendung nach einem der Ansprüche 1 bis 8, formuliert zur oralen Verabreichung.

10. Verbindung oder Salz zur Verwendung nach Anspruch 1, wobei die Krebserkrankung gegen einen EGFR-Inhibitor resistent ist und **gekennzeichnet ist durch** die Expression von mindestens einer KRAS-Mutation, die ausgewählt ist aus der Gruppe bestehend aus G12D, G12V und G13D; und gegebenenfalls einer EGFR-Mutation, die ausgewählt ist aus der Gruppe bestehend aus L858R, T790M, C797S und einer Kombination davon.

11. Verbindung oder Salz zur Verwendung nach Anspruch 10, wobei die Krebserkrankung eine durch KRAS ausgelöste Krebserkrankung ist und die Verbindung oder das Salz in einer Dosierung von 1-500 mg/kg oral verabreicht wird.

12. Verbindung oder Salz zur Verwendung nach Anspruch 11, wobei die Krebserkrankung ausgewählt ist aus der Gruppe bestehend aus Pankreaskrebs, Dickdarmkrebs, Kopf- und Halskrebs und Lungenkrebs und die Verbindung oder das Salz in einer Dosierung von 20-40 mg/kg oral verabreicht wird.

## Revendications

1. Composé de formule I ou sel pharmaceutiquement acceptable de celui-ci : 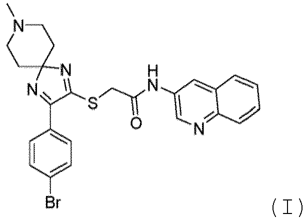 à utiliser pour le traitement d'un cancer **caractérisé par** l'expression d'au moins une mutation KRAS, dans lequel le composé de formule I modifie l'activité de KRAS ou de cMet résultant de ladite mutation KRAS.

2. Composé ou sel à utiliser selon la revendication 1, dans lequel le cancer est un cancer induit par KRAS.

3. Composé ou sel à utiliser selon la revendication 2, dans lequel le cancer est choisi dans le groupe constitué par le cancer du pancréas, le cancer colorectal, le cancer de la tête et du cou et le cancer du poumon.

4. Composé ou sel à utiliser selon l'une quelconque des revendications 1 à 3, dans lequel le cancer est **caractérisé par** l'expression d'au moins une mutation KRAS choisie dans le groupe constitué de G12D, G12V et G13D et, éventuellement, d'une mutation EGFR choisie dans le groupe constitué de L858R, T790M, C797S, S768l, del Exon 19 et d'une combinaison de celles-ci, de préférence dans laquelle la mutation KRAS est G12D ou G13D.

5. Composé ou sel à utiliser selon la revendication 4, dans lequel la mutation de l'EGFR est L858R ou T790M.

6. Composé ou sel à utiliser selon l'une quelconque des revendications 1 à 5, dans lequel le cancer est résistant à un inhibiteur de l'EGFR.

7. Composé ou sel à utiliser selon la revendication 6, dans lequel l'inhibiteur de l'EGFR est le cetuximab ou l'osimertinib.

8. Composé ou sel à utiliser selon l'une quelconque des revendications 1 à 7, formulé pour être administré à une dose de 1 à 500 mg/kg.

9. Composé ou sel à utiliser selon l'une quelconque des revendications 1 à 8, formulé pour être administré par voie orale.

10. Composé ou sel à utiliser selon la revendication 1, dans lequel le cancer est résistant à un inhibiteur de l'EGFR et est **caractérisé par** l'expression d'au moins une mutation KRAS choisie dans le groupe constitué par G12D, G12V et G13D ; et, éventuellement, une mutation EGFR choisie dans le groupe constitué par L858R, T790M, C797S et une combinaison de celles-ci.

11. Composé ou sel à utiliser selon la revendication 10, dans lequel le cancer est un cancer induit par KRAS et le composé ou le sel est administré par voie orale à une dose de 1 à 500 mg/kg.

12. Composé ou sel à utiliser selon la revendication 11, dans lequel le cancer est choisi dans le groupe constitué par le cancer du pancréas, le cancer colorectal, le cancer de la tête et du cou et le cancer du poumon, et dans lequel le composé ou le sel est administré par voie orale à une dose de 20 à 40 mg/kg.
